# EUROPEAN PATENT APPLICATION

(11) **EP 4 378 456 A1**
(43) Date of publication of application: **05.06.2024**
(21) Application number: 21951329.8
(22) Date of filing: 30.07.2021
(51) Int. Cl.: A61K 9/22, A61K 31/52, A61K 47/58, A61P 25/00

(54) **CONTROLLED-RELEASE ORAL PREPARATION AND PREPARATION METHOD THEREFOR**

(71) Applicant: Panion & BF Biotech Inc., Taipei (TW)
(72) Inventor: CHUANG, Jwey Yuan, Taipei, Taiwan 115 (TW); SU, Yu De, Taipei, Taiwan 115 (TW); LU, Shao Chuan, Taipei, Taiwan 115 (TW)
(74) Representative: Becker, Eberhard
(86) International application number: PCT/CN2021/109543
(87) International publication number: WO 2023/004733

(57) **Abstract**

A two-layer controlled-release oral preparation, which comprises a first ingredient as a quick-release layer and a second ingredient as a controlled-release layer, wherein the first ingredient comprises a xanthine derivative, and the second ingredient comprises a xanthine derivative and an enteric excipient. No more than 35% by weight of the xanthine derivative in the second ingredient is released within 90 min after in vitro elution, and 50% or more by weight of the xanthine derivative in the second ingredient is released within 3 h after in vitro elution.

## Description

### Technical Field

The present invention relates to an oral formulation, and in particular, relates to a controlled-release oral formulation and preparation method thereof.

### Related Art

Caffeine is a xanthine derivative, and is a bipolar substance that is soluble in water and lipids, and its chemical structure is very similar to adenosine. Adenosine is a skeleton of adenosine triphosphate (ATP), which is the body's energy source, and it is also a neuromodulator. In addition to reducing wakefulness and alertness, adenosine and adenosine compounds reduce the concentration of excitatory neurotransmitters. Caffeine has a similar structure to adenosine and competes with adenosine for receptors, thus caffeine has an invigorating effect. On the other hand, caffeine can reduce the sensation of muscles' pain during exercise, thereby achieving an anti-fatigue effect.

According to reports, approximately 74% of elite athletes use caffeine as a supplement before or during competition, with endurance exercise athletes showing a higher prevalence of use. Possible reasons for caffeine's popularity may be its easy availability, low side effects, and proven ergogenic effects can be achieved at low doses (<6 mg/kg).

In general, an absorption rate of caffeine reaches its highest plasma concentration within 30 minutes to 90 minutes after ingestion. Therefore, according to the results from many clinical trials, it is recommended that users should take caffeine 30-60 minutes before exercise to obtain a caffeine boosting effect during the competition. For endurance exercises, such as marathons, an average completion time is around 4.5 hours. When caffeine is consumed 30-60 minutes before the competition, individuals will often reach a lower plasma caffeine state after about 3 hours to 3.5 hours into the event which is beyond caffeine's half life. This is also the high-pressure period or bonking period of the competition. Therefore, in order to avoid the feeling of fatigue or perception of exertion caused by the bonking period, many users will ingest low to medium doses of caffeine (~3 mg/kg) in the middle and late stages of the competition (with 1 to 2 hours left to finish) for supplementation.

However, endurance exercise requires multiple supplements such as carbohydrate energy gels, electrolytes, amino acids, etc., and additionally carrying and consuming caffeine during the exercise becomes very inconvenient. In addition, a single dose of caffeine still requires about an hour for absorption, which is not as useful as sustained release formulations in providing a long-lasting caffeine boost. In addition, for sustained-release formulations of caffeine, the technology involves gradual degradation of polymers in the body for slower release of caffeine. However, it lacks specific release segments in the human gastrointestinal tract, making it unable to meet the controlled-release requirements to achieve the goal of providing sufficient caffeine release within a specific time period after ingestion. This limitation prevents the maintenance of elevated plasma caffeine levels throughout the entire exercise period, thereby compromising the enhancement of exercise performance.

Accordingly, how to effectively control the release of xanthine derivatives to maintain its sufficient plasma concentrations, to resolve the necessities of repeated supplementations/stimulations during prolonged activities, such as during endurance sports like marathons where runners need to take additional caffeine products for performance enhancement, or situations like long distance driving where inconvenience and distraction can be caused by repeated supplementation, is currently a problem that needs to be resolved.

### SUMMARY OF INVENTION

The present invention provides an oral formulation that can effectively control the release of xanthine derivatives to maintain high plasma concentrations, thereby meeting sustained and long-term ergogenic effect/stimulations demands. For example, the oral formulation of the present invention can avoid the distraction from the need to take and ingest caffeine during endurance exercise, and a high caffeine content/high plasma concentration can be maintained during the entire exercising period.

An oral formulation of the present invention includes a first composition and a second composition. The first composition includes a xanthine derivative. The second composition includes a xanthine derivative and an enteric excipient. The in vitro dissolution of the second composition releases less than 35% by weight of the xanthine derivative in the second composition within 90 minutes, and the in vitro dissolution of the second composition releases 50% or more by weight of the xanthine derivative in the second composition within 3 hours.

In an embodiment of the present invention, the in vitro dissolution of the second composition releases 50% by weight or more to 70% by weight or less of the xanthine derivative in the second composition within 3 hours.

In an embodiment of the present invention, the in vitro dissolution of the first composition releases 80% by weight or more of the xanthine derivative in the first composition within 1 hour.

In an embodiment of the present invention, the in vitro dissolution of the first composition releases up to 100 % by weight of the xanthine derivative in the first composition within 90 minutes, the in vitro dissolution of the second composition releases 90% or more by weight of the xanthine derivative in the second composition within 8 hours to 12 hours, and the in vitro dissolution of the second composition releases up to 100 % by weight of the xanthine derivative in the second composition within 12 hours to 18 hours.

In an embodiment of the present invention, the in vitro dissolution of the second composition releases less than 40% by weight of the xanthine derivative in the second composition within 2 hours.

In an embodiment of the present invention, the oral formulation is an oral bilayer tablet, which includes a first layer composed of the first composition, and a second layer composed of the second composition.

In an embodiment of the present invention, a weight ratio of the first composition to the second composition is 1: 1 to 1:1.2.

In an embodiment of the present invention, the enteric excipient is a zein enteric coating.

In an embodiment of the present invention, when a weight proportion of the second composition is 100%, a weight proportion of the zein enteric coating is 5.5% to 10% of the second composition.

In an embodiment of the present invention, a total weight of the xanthine derivative in the oral formulation is 50mg to 400mg.

In an embodiment of the present invention, a total weight of the xanthine derivative in the first composition is 25mg to 200mg.

In an embodiment of the present invention, a total weight of the xanthine derivative in the second composition is 25mg to 200mg.

In an embodiment of the present invention, the first composition further includes green tea extract, polyvinylpyrrolidone, microcrystalline cellulose, crospovidone, magnesium stearate and silicon dioxide, wherein the green tea extract includes the xanthine derivative.

In an embodiment of the present invention, the second composition further comprises green tea extract, polyvinylpyrrolidone, microcrystalline cellulose, medium chain triglyceride, magnesium stearate, silicon dioxide and coating 145K290001 (Nutrafinish ^{®} Dietary Supplement Coating 145K290001 Clear (NSC)), wherein the green tea extract includes the xanthine derivative.

In an embodiment of the present invention, the oral formulation reaches the highest plasma concentration of the xanthine derivative within 90 minutes of ingestion.

In a preparation method of the oral formulation of the present invention, the method includes: forming a bilayer tablet with a powder of the first composition and a powder of the second composition.

In an embodiment of the present invention, a preparation of the powder of the second composition includes the following steps. Providing a coating suspension including the enteric excipient. Providing a green tea extract, wherein the green tea extract includes the xanthine derivative. Spray granulating the green tea extract with the coating suspension so as to gain weight in a range of 5% to 15%.

In an embodiment of the present invention, the method further includes: after spray granulation, performing drying and sieving to form the powder of the second composition.

In an embodiment of the present invention, a preparation of the powder of the first composition includes the following steps. Providing a green tea extract, wherein the green tea extract includes the xanthine derivative. Adding an alcohol aqueous solution of polyvinylpyrrolidone to a powder including the green tea extract, and after granulation, performing drying and sieving to form the powder of the first composition.

A method of using the oral formulation of the present invention includes: ingesting the oral formulation 30 minutes to 60 minutes before performing endurance exercise, and a dosage of the oral formulation is 3mg/kg to 6mg/kg.

Based on the above, by using an oral formulation having a first composition and a second composition according to the embodiments of the present invention, the first composition can be quickly released at an early stage after ingestion to provide an early caffeine supplementation, and the caffeine of the second composition can be controlled released at a later stage.

Accordingly, the bilayer controlled-release oral formulation of the present invention can effectively control the release of xanthine derivatives (such as caffeine) to maintain high plasma concentrations, thereby meeting sustained and long-term ergogenic effects/stimulation demands. For example, the oral formulation of the present invention can avoid the distraction from the need to take and ingest caffeine during endurance exercise, and through the controlled release of the second composition, the time of maintaining a high caffeine content/high plasma concentration can be extended throughout the entire exercising period. In addition, the oral formulation of the present invention can also avoid the inconvenience or distraction from the need to supplement caffeine during long distance driving.

### BRIEF DESCRIPTION OF DRAWINGS

The accompanying drawings are included to provide a further understanding of the invention, and are incorporated in and constitute a part of this specification. The drawings illustrate embodiments of the invention and, together with the description, serve to explain the principles of the invention.
FIG. 1 is a schematic diagram of an oral formulation according to an embodiment of the present invention;
FIG. 2 is a flow chart for preparing a first composition powder according to an embodiment of the present invention;
FIG. 3 is a flow chart for preparing a second composition powder according to an embodiment of the present invention;
FIG. 4 is a flow chart for preparing a sustained release powder according to a comparative example of the present invention;
FIG. 5 is an in vitro dissolution profile diagram of a second composition according to the embodiment of the present invention and a comparative example;
FIG. 6 is an in vitro dissolution profile comparison diagram of an enteric granulation of the present invention and a conventional matrix type granulation;
FIG. 7 is an in vitro dissolution profile diagram of a first composition according to an embodiment of the present invention;
FIG. 8 is an in vitro dissolution profile diagram of an oral formulation according to an embodiment of the present invention.

Description of reference numerals:
100: oral formulation;
102: first layer;
102A: first composition powder;
104: second layer;
104A: second composition powder;
A10, A12, A14, A16, A18, A20, A22, A24, A26, A28, A30, A32: steps;
B 10, B12, B14, B16, B18, B20, B22, B24, B26, B28, B30, B32, B34, B36: steps;
C10, C12, C14, C16, C18, C20, C22, C24, C26, C28, C30, C32, C34, C36, C38: steps.

### DESCRIPTION OF EMBODIMENTS

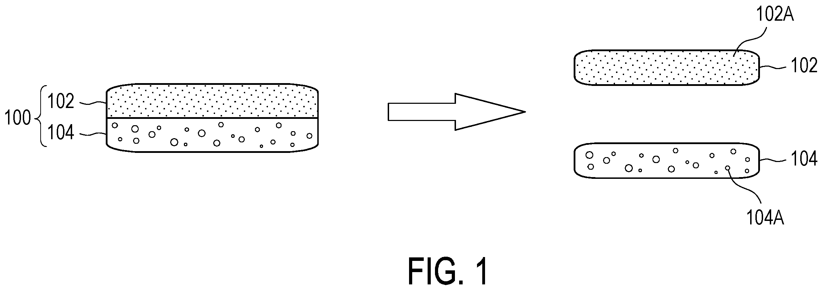

FIG. 1 is a schematic diagram of an oral formulation according to an embodiment of the present invention. Referring to FIG. 1, the oral formulation 100 according to the embodiments of the present invention comprises a first layer 102 composed of a first composition and a second layer 104 composed of a second composition. For example, the first layer 102 includes a first composition powder 102A, and the second layer 104 includes a second composition powder 104A. In some embodiments, a preparation method of the oral formulation 100 includes preparing the first composition powder 102A and the second composition powder 104A into a bilayer tablet using a bilayer tableting machine to form the oral formulation 100. Although the oral formulation 100 shown in FIG. 1 uses an oral bilayer tablet as an example, the present invention is not limited thereto.

In some other embodiments, the oral formulation 100 may also include other types of dosage forms and preparation methods. In an embodiment, the preparation of the oral formulation 100 includes separately granulating the first composition powder 102A and the second composition powder 104A, and then filling into capsules in the form of pellets. In an embodiment, the preparation of the oral formulation 100 includes separately granulating the first composition powder 102A and the second composition powder 104A, and then filling into sachet in the form of pellets. In another embodiment, the preparation of the oral formulation 100 includes separately tableting the first composition powder 102A and the second composition powder 104A, and then filling into capsules in the form of micro-tablets/mini-tablets. In yet another embodiment, the preparation of the oral formulation 100 includes separately tableting the first composition powder 102A and the second composition powder 104A, and then filling into sachet in the form of micro-tablets/mini-tablets.

It can be understood from the above description that the oral formulation 100 of the present invention is not limited to a bilayer tablet dosage form. For example, as long as the oral formulation 100 includes a first composition powder 102A as an immediate release layer and a second composition powder 104A as a controlled-release layer, a dosage form of the oral formulation 100 can be appropriately modified. In an embodiment, the second composition powder 104A serves as a delayed release layer. The first composition/first composition powder 102A and the second composition/second composition powder 104A will be further described below.

In an embodiment of the present invention, the first composition/first composition powder 102A may at least include green tea extract, polyvinylpyrrolidone, microcrystalline cellulose, crospovidone, magnesium stearate and silicon dioxide. The green tea extract can include xanthine derivatives (or caffeine). In some embodiments, microcrystalline cellulose can be replaced with lactose or corn starch. Crospovidone can be replaced with sodium carboxymethyl starch, croscarmellose sodium, hydroxypropyl cellulose, and pregelatinized starch.

In an embodiment, the preparation of the first composition powder 102A may include the following steps, but is not limited thereto. Firstly, green tea extracts including xanthine derivatives are provided. The green tea extracts, microcrystalline cellulose and crospovidone are sieved and de-agglomerated, mixed evenly and placed into a super mixer granulator. Subsequently, an alcohol aqueous solution of polyvinylpyrrolidone is added to the above-mentioned powder including the green tea extracts, and the granulation is performed by a granulator, followed by drying, sieving and de-agglomeration. Next, crospovidone, microcrystalline cellulose and silicon dioxide are added and mixed evenly to form the first composition powder 102A. Accordingly, the obtained first composition powder 102A can be used to form the above-mentioned oral formulation 100 in different dosage forms.

In an embodiment of the present invention, the second composition/second composition powder 104A may include green tea extracts, enteric excipients, polyvinylpyrrolidone, microcrystalline cellulose, medium chain triglyceride, magnesium stearate, silicon dioxide, and coating 145K290001 (Nutrafinish ^{®} Dietary Supplement Coating 145K290001 Clear (NSC)). The green tea extracts can include xanthine derivatives (or caffeine). The coating 145K290001 is a mixture of hydroxypropyl methyl cellulose, triacetin and talc. The enteric excipient can be zein enteric coating.

In some embodiments, microcrystalline cellulose can be replaced with lactose or corn starch. Medium chain triglyceride can be replaced with glycerol, polyethylene glycol (PEG), mineral oil or fatty acid. In addition, in some embodiments, in addition to zein enteric coating, the enteric excipients can also be ethyl acrylate/methacrylic acid copolymer, hydroxypropyl methylcellulose acetate succinate, cellulose acetate phthalate or hydroxypropyl methylcellulose phthalate, the like, or mixtures thereof.

In an embodiment, the preparation of the second composition powder 104A may include the following steps, but is not limited thereto. Firstly, an appropriate amount of alcohol aqueous solution is weighed, medium chain triglyceride is added while stirring, and zein as an enteric excipient and coating 145K290001 are mixed and slowly added to the alcohol aqueous solution, and the components are completely dissolved to obtain a coating suspension. Subsequently, green tea extracts including xanthine derivatives are provided. The green tea extract is spray granulated with the coating suspension so as to gain weight in a range of 5% to 15%, and drying, sieving and de-agglomeration are then performed. Thereafter, microcrystalline cellulose, polyvinylpyrrolidone, magnesium stearate and silicon dioxide are added and mixed evenly to form the second composition powder 104A. Accordingly, the obtained second composition powder 104A can be used to form the above-mentioned oral formulation 100 in different dosage forms.

In an embodiment of the present invention, a total weight of the xanthine derivative (caffeine) in the oral formulation 100 is 50 mg to 400 mg. For example, a weight of the xanthine derivative in the first composition is 25 mg to 200 mg, and a weight of the xanthine derivative in the second composition is 25 mg to 200 mg. In some embodiments, a weight ratio of the first composition to the second composition in the oral formulation 100 is 1: 1 to 1:1.2. In addition, when a weight proportion of the second composition is 100%, a weight proportion of the zein enteric coating as the enteric excipient is 5.5% to 10% of the second composition. When the weight proportion of the zein enteric coating is controlled within the above range, a more ideal controlled-release effect can be achieved, especially by using enteric material coating to reduce the release in the early stage, followed by release upon reaching the intestines.

In the oral formulation 100 of the present invention, the in vitro dissolution of the first composition releases 80% by weight or more of the xanthine derivative in the first composition within 1 hour. The in vitro dissolution of the first composition releases up to 100% by weight of the xanthine derivative in the first composition within 90 minutes. In addition, the in vitro dissolution of the second composition releases less than 35% by weight of the xanthine derivative in the second composition within 90 minutes, and the in vitro dissolution of the second composition releases 50% by weight or more of the xanthine derivative in the second composition within 3 hours. In some embodiments, the in vitro dissolution of the second composition releases 50% by weight or more to 70% by weight or less of the xanthine derivative in the second composition within 3 hours. In addition, the in vitro dissolution of the second composition releases less than 40% by weight of the xanthine derivative in the second composition within 2 hours, while the in vitro dissolution of the second composition releases 90% or more by weight or more of the xanthine derivative in the second composition within 8 hours to 12 hours, and the in vitro dissolution of the second composition releases up to 100 % by weight of the xanthine derivative in the second composition within 12 hours to 18 hours. Accordingly, in the oral formulation 100 of the present invention, the first composition (the first composition powder 102A) can be used as an immediate release layer, and the second composition (the second composition powder 104A) can be used as a controlled-release layer, so that a bilayer controlled release oral formulation 100 with immediate release at an early stage and controlled release at a later stage (or delayed release) can be achieved.

In an embodiment of the present invention, the in vitro dissolution data of xanthine derivatives can be used to simulate/correspond to the results of its in vivo dissolution. In some embodiments, the in vitro dissolution test method includes using a paddle apparatus (i.e., USP apparatus II) to perform the test at 50 rpm to 75 rpm at a temperature of 37±0.5°C. The oral formulations are tested in at least three buffer solutions that are capable of simulating the gastrointestinal tract (pH range from 1.2 to 6.8). For example, when conducting a dissolution test with a total test time of 4 hours, the test is performed in a pHH 1.2 buffer solution in the 0-1 hour time period, replaced with a pHH 4.5 buffer solution during the 1-2 hours time period, and replaced with a pH 6.8 buffer solution during the 2-4 hours time period. More specifically, the pH 1.2 buffer solution is used to simulate a gastric fluid environment, the pH 4.5 buffer solution is used to simulate a duodenal fluid environment, and the pH 6.8 buffer solution is used to simulate an intestinal fluid environment. In the experiments, solutions were collected at 30 minutes, 60 minutes, 90 minutes, 120 minutes, 150 minutes, 180 minutes and 240 minutes after the start of dissolution, and the dissolution percentage was calculated based on the standard.

In some embodiments of the present invention, the oral formulation 100 reaches the highest plasma concentration of the xanthine derivative (caffeine) within 90 minutes of ingestion. When the oral formulation 100 is used as a supplement for endurance exercise, a method of using the oral formulation 100 includes ingesting the oral formulation 100 within 30 minutes to 60 minutes before performing endurance exercises, and an ingestion dose of the oral formulation 100 is calculated based on a body weight of the user, which is 3 mg/kg (3 milligram per kilogram) to 6 mg/kg. After ingesting the oral formulation 100 of the present invention, the interference of having to additionally take and ingest caffeine during endurance exercise can be avoided, and by using the controlled-release second composition, a high content/high plasma concentration of caffeine can be maintained for at least 6 hours to 8 hours.

In some embodiments, endurance exercise athletes may ingest the oral formulation 100 of the present invention 30 minutes before the competition. For example, if a weight of an athlete is 60 kg, then approximately 180 mg to 360 mg of the oral formulation 100 is ingested. In addition, a weight ratio of the first composition to the second composition in the oral formulation 100 is approximately 1:1 to 1:1.2. Within 1 hour to 2 hours after ingesting the oral formulation 100, since the first composition (immediate release layer) of the oral formulation 100 has released 80% by weight or more of the xanthine derivative (caffeine), the user can maintain high plasma concentrations of the xanthine derivative during the early stages of endurance exercise (up to 2 hours from the start) to provide a supplementing effect.

During a later stage of endurance exercise, such as within 2 hours to 4.5 hours after ingesting the oral formulation 100, since the first composition has been fully released and has entered the half-life stage, therefore, the plasma concentration of the xanthine derivative (caffeine) from the first composition will slowly decrease. In comparison, the xanthine derivative (caffeine) of the second composition (controlled-release layer) of the oral formulation 100 releases less than 35% by weight during early stages, such as within 90 minutes after ingestion, and will release 50% by weight or more to 70% by weight or less within 3 hours after ingestion. As such, the second composition (controlled release layer) can increase and maintain high plasma concentrations of the xanthine derivative (caffeine) at later stages of the endurance exercise (2 hours to 4.5 hours), and will provide the endurance exercise athletes with a second dose of caffeine boost effect.

Accordingly, endurance exercise athletes can maintain high plasma concentrations of the xanthine derivatives (caffeine) throughout the endurance exercise period to promote/support their exercise-related performance. In addition, since the xanthine derivative (caffeine) in the second composition is released up to 100% by weight within 12 hours to 18 hours after ingestion, even after the end of the endurance exercise, during the continuous release period of the caffeine, the ergogenic/stimulating effects can be continuously prolonged and maintained.

### Examples

To prove the difference between the in vitro dissolution effect of the oral formulation 100 of the present invention and the conventional matrix type sustained release dosage form, the following examples are performed and described in detail. Hereinafter, the preparation of the first composition powder 102A, the second composition powder 104A and the conventional matrix-type sustained release powder will be described.

### Preparation example of the first composition powder

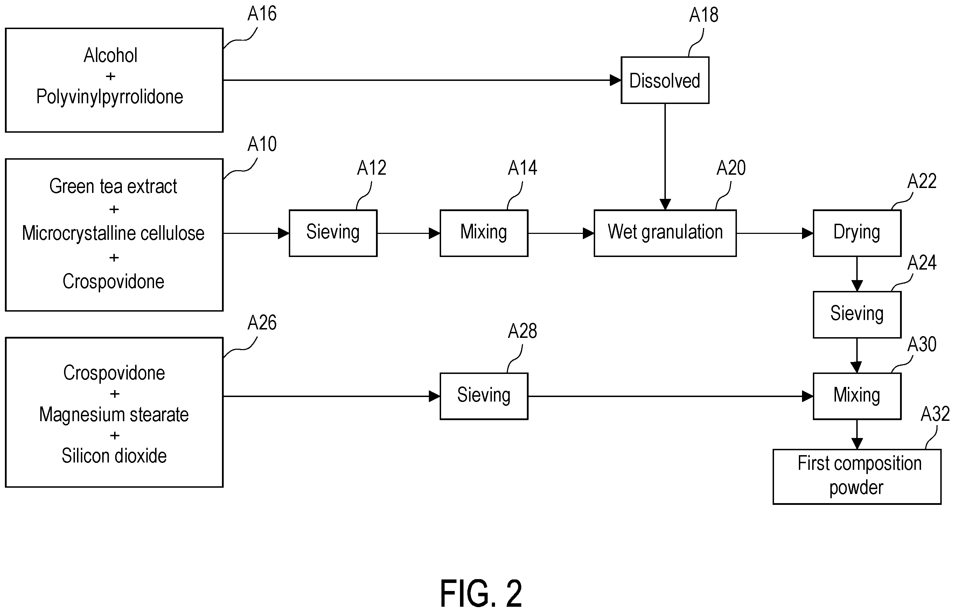

FIG. 2 is a flow chart for preparing a first composition powder according to an embodiment of the present invention. The various materials used in FIG. 2 are prepared according to the contents in Preparation Examples A1 to A5 as shown in Table 1.

**Table 1: Formulations of the first composition powder**

| First Composition | | Preparation Example A1 | Preparation Example A 2 | Preparation Example A3 | Preparation Example A4 | Preparation Example A5 |
|---|---|---|---|---|---|---|
| | Material name | Proportion (%) | Proportion (%) | Proportion (%) | Proportion (%) | Proportion (%) |
| 1 | Alcohol aqueous solution 95% | - | - | - | - | - |
| 2 | Green tea extract | 78.13% | 78.13% | 83.33% | 83.33% | 83.33% |
| 3 | Microcrystalline cellulose | 7.18% | 7.18% | 7.97% | 7.97% | 7.97% |
| 4 | Crospovidone | 10.00% | 10.00% | 4.00% | 4.00% | 4.00% |
| 5 | Polyvinylpyrrolidone | 3.00% | 3.00% | 3.00% | 3.00% | 3.00% |
| 6 | Magnesium stearate | 1.50% | 1.50% | 1.50% | 1.50% | 1.50% |
| 7 | Silicon dioxide | 0.20% | 0.20% | 0.20% | 0.20% | 0.20% |
| | | 100.00% (320mg) | 100.00% (320mg) | 100.00% (300mg) | 100.00% (300mg) | 100.00% (300mg) |

Referring to step A10 in FIG. 2 and Table 1, firstly, green tea extract, microcrystalline cellulose and crospovidone are mixed and prepared. Subsequently, after sieving and de-agglomeration as in step A12, the above components are mixed evenly in step A14 and placed into a super mixer granulator. Thereafter, as in step A16, polyvinylpyrrolidone is added into a 95% alcohol aqueous solution, and stirred in step A18 until it is completely dissolved. Next, referring to step A20, the solution obtained in step A18 is added to the powder in step A14, and wet granulation is performed using the super mixer granulator. After the wet granulation is completed, drying is performed in step A22, and sieving and de-agglomeration are performed in step A24. Thereafter, in step A26, crospovidone, magnesium stearate and silicon dioxide are prepared in amounts as listed in Table 1, and sieving is then performed in step A28. Next, in step A30, the powder obtained in step A28 and the powder obtained in step A24 are mixed. Accordingly, a first composition powder can be obtained in step A32.

### Preparation example of the second composition powder

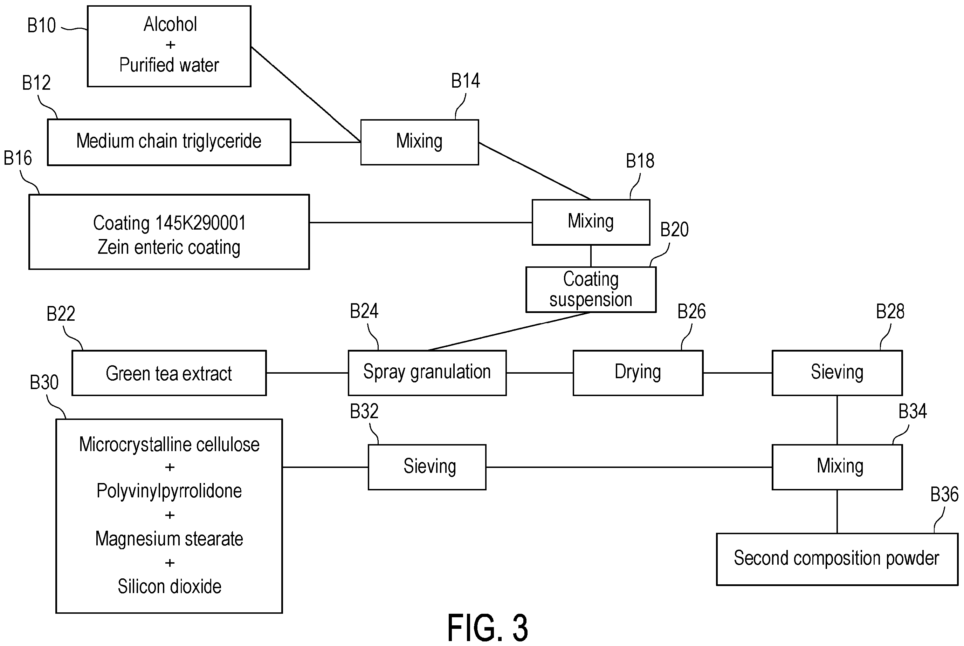

FIG. 3 is a flow chart for preparing a second composition powder according to an embodiment of the present invention. The various materials used in FIG. 3 are prepared according to the contents in Preparation Examples B 1 to B4 as shown in Table 2.

**Table 2: Formulations of the second composition powder**

| Second Composition | | Preparation Example B 1 (Ex 1) | Preparation Example B2 (Ex 2) | Preparation Example B3 (Ex 3) | Preparation Example B4 (Ex 4) |
|---|---|---|---|---|---|
| | Material name | Proportion (%) | Proportion (%) | Proportion (%) | Proportion (%) |
| 1 | Alcohol aqueous solution 95% | - | - | - | - |
| 2 | Purified water | - | - | - | - |
| 3 | Green tea extract | 71.43% | 71.43% | 71.43% | 71.43% |
| 4 | Microcrystalline cellulose | 16.00% | 13.14% | 22.64% | 19.43% |
| 5 | Zein enteric coating | 8.00% | 10.00% | 3.00% | 5.50% |
| 6 | Medium chain triglyceride | 1.64% | 2.14% | 0.64% | 1.07% |
| 7 | Coating 145K290001 | 1.07% | 1.43% | 0.43% | 0.71% |
| 8 | Polyvinylpyrrolidone | 0.36% | 0.36% | 0.36% | 0.36% |
| 9 | Magnesium stearate | 1.00% | 1.00% | 1.00% | 1.00% |
| 10 | Silicon dioxide | 0.50% | 0.50% | 0.50% | 0.50% |
| | | 100.00% (350mg) | 100.00% (350mg) | 100.00% (350mg) | 100.00% (350mg) |

Referring to step B10 in FIG. 3 and Table 2, purified water and alcohol are mixed to prepare an alcohol aqueous solution. Next, in step B12, medium-chain triglyceride is provided, and in step B14, the medium-chain triglyceride is added to the aqueous alcohol solution while stirring and mixed. Subsequently, referring to step B 16, after mixing the zein enteric coating and coating 145K290001, the mixed components are slowly added to the above alcohol aqueous solution for mixing in step B18. In step B20, the components added to the alcohol aqueous solution are stirred until they are completely dissolved and dispersed to form a coating suspension. After obtaining the coating suspension, a green tea extract is provided in step 22, and the green tea extract is spray granulated in step B24 with the coating suspension obtained in step B20. In step B24, the Yenchen's fluid bed granulator (model FBDD-2) is used for spray granulation, and the parameters are as shown in Table 3.

**Table 3: Spray granulation parameters**

| Inlet air (°C) | Outlet air (°C) | Product temperature (°C) | Exhaust air(%) | Flow rate (rpm) | Vibration (sec/sec) | Fan(rpm) | Pipe diameter (Ø) |
|---|---|---|---|---|---|---|---|
| 45-60 | 32-42 | 31-38 | 100 | 5-25 | 5/55 | 1500-2500 | 6 |

After spray granulation, drying is performed in step B26, and sieving and de-agglomeration are performed in step B28. Thereafter, in step B30, microcrystalline cellulose, polyvinylpyrrolidone, magnesium stearate and silicon dioxide are prepared according to the contents shown in Table 2, and then sieving is performed in step B32. Next, in step B34, the powder obtained in step B28 and the powder obtained in step B32 are mixed. Accordingly, a second composition powder can be obtained in step B36.

### Preparation example of conventional sustained release powder

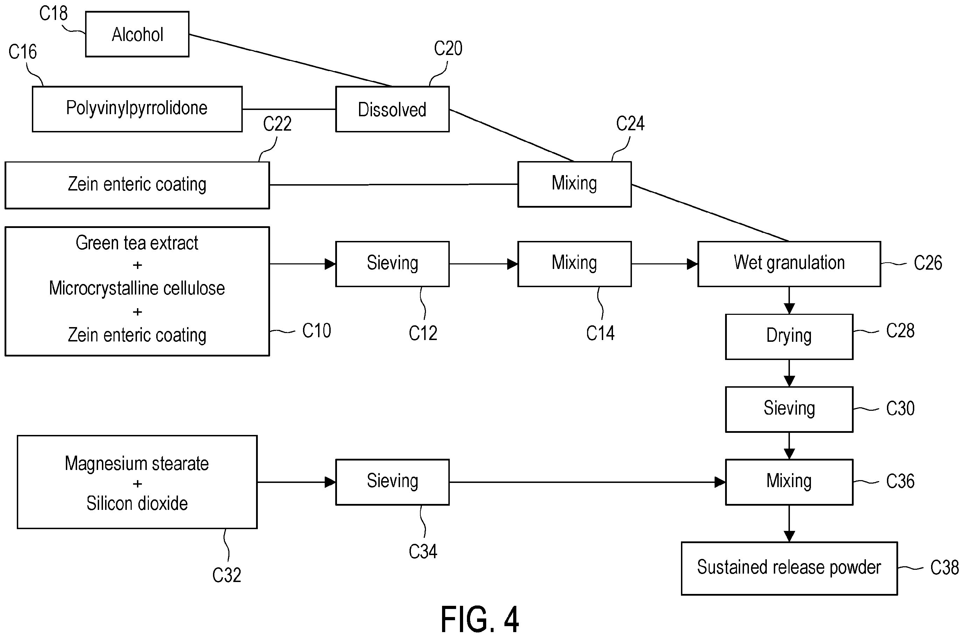

FIG. 4 is a flow chart for preparing a sustained release powder according to a comparative example of the present invention. The various materials used in FIG. 4 are prepared according to the contents in Preparation Example C5 as shown in Table 4.

**Table 4: Formulations of the sustained release powder**

| Sustained release powder | | Preparation Example C5 (Ex 5) |
|---|---|---|
| | Material name | Proportion (%) |
| 1 | Alcohol aqueous solution 95% | - |
| 2 | Purified water | - |
| 3 | Green tea extract | 71.43% |
| 4 | Microcrystalline cellulose | 16.07% |
| 5 | Zein enteric coating | 10.00% |
| 6 | Medium chain triglyceride | 0.00% |
| 7 | Coating145K290001 | 0.00% |
| 8 | Polyvinylpyrrolidone | 1.00% |
| 9 | Magnesium stearate | 1.00% |
| 10 | Silicon dioxide | 0.50% |
| | | 100.00% (350mg) |

Referring to step C10 of FIG. 4 and Table 4, green tea extract, microcrystalline cellulose and zein enteric coating are mixed together. subsequently, after sieving and de-agglomeration in step C12, the above components are mixed evenly in step C14 and then placed into a super mixer granulator. Thereafter, polyvinylpyrrolidone and 95% alcohol aqueous solution are provided in step C16 and step C18. Furthermore, the polyvinylpyrrolidone is added to the 95% alcohol aqueous solution in step C20, and mixed and stirred until it is completely dissolved. In step C22, zein enteric coating is provided, and the zein enteric coating is added to the above alcohol aqueous solution in step C24, and mixed to suspend and disperse therein. Referring to step C26, the solution obtained in step C24 is added to the powder in step C14, and wet granulation is performed with the super mixer granulator. After wet granulation, drying is performed in step C28, and sieving and de-agglomeration are performed in step C30. Next, in step C32, magnesium stearate and silicon dioxide are prepared in amounts as listed in Table 4, and then sieving is performed in step C34. Next, in step C36, the powder obtained in step C30 and the powder obtained in step C34 are mixed. Accordingly, conventional sustained release powder can be obtained in step C38.

### In vitro dissolution test I

To confirm the difference in caffeine release between the second composition of the present invention (Preparation Examples B1 to B4) and the conventional sustained release powder (Preparation Example C5), an in vitro dissolution test was conducted in the following manner.

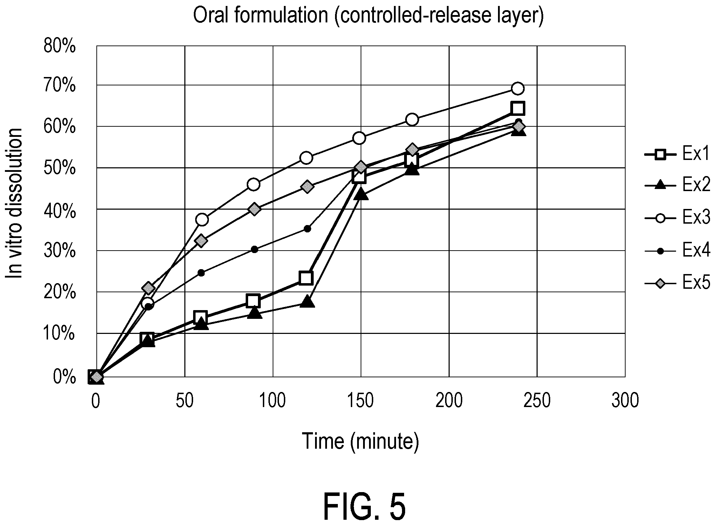

In the in vitro dissolution test of the current experimental example, a paddle apparatus (i.e., USP apparatus II) was used to conduct the test at 50 rpm and at a temperature of 37±0.5°C. The second composition of the present invention (Preparation Examples B1 to B4) and the conventional sustained release powder (Preparation Example C5) are placed in a simulated gastric fluid of pHH 1.2 during the 0-1 hour time period, placed in a simulated duodenal fluid at pHH 4.5 during the 1-2 hours time period, and placed in simulated intestinal fluid at pH 6.8 during the 2-4 hours time period. In the above, a certain amount of solution was collected at different time points (30 minutes, 60 minutes, 90 minutes, 120 minutes, 150 minutes, 180 minutes and 240 minutes) for calculating a concentration of xanthine derivatives. Herein, an ultraviolet-visible spectrometer (UV/VIS) is used to perform concentration analysis, and the dissolution percentage was calculated and confirmed based on the standard. The test results are shown in FIG. 5 and Table 5.

**Table 5: In vitro dissolution of the second composition (controlled-release layer) and the comparative example**

| | Time (minute) | Preparation Example B 1 (Ex 1) | Preparation example B2 (Ex 2) | Preparation example B3 (Ex 3) | Preparation example B4 (Ex 4) | Preparation example C5 (Ex 5) |
|---|---|---|---|---|---|---|
| pH 1.2 | 0 | 0.0% | 0.0% | 0.0% | 0.0% | 0.0% |
| | 30 | 8.7% | 8.5% | 17.4% | 17.0% | 21.6% |
| | 60 | 14.1% | 12.6% | 38.1% | 25.9% | 33.0% |
| pH 4.5 | 90 | 18.2% | 15.4% | 46.4% | 30.7% | 40.4% |
| | 120 | 23.5% | 17.9% | 52.9% | 35.8% | 46.0% |
| pH 6.8 | 150 | 48.1% | 44.1% | 57.7% | 50.1% | 50.6% |
| | 180 | 52.3% | 50.1% | 62.2% | 55.2% | 54.5% |
| | 240 | 64.3% | 59.8% | 69.3% | 61.5% | 60.7% |

FIG. 5 is an in vitro dissolution profile diagram of a second composition according to the embodiment of the present invention and a comparative example. Referring to FIG. 5 and Table 5, it can be seen that Preparation Example B1 (Ex1) and Preparation Example B2 (Ex2) have better controlled-release effects. Wherein, within 2 hours of in vitro dissolution, the release of xanthine derivatives (caffeine) are less than 30% by weight, and are only 23.5% by weight and 17.9% by weight respectively. Moreover, the xanthine derivatives (caffeine) of Preparation Example B1 (Ex1) and Preparation Example B2 (Ex2) are released up to 48.1% by weight and 44.1% by weight respectively after 2.5 hours. Therefore, the second composition prepared in Preparation Example B1 (Ex1) and Preparation Example B2 (Ex2) can start to elicit a noticeable xanthine derivative (caffeine) boosting effect after 2 hours, and thus will only undergo controlled release upon entering the intestinal environment (pH 6.8). Accordingly, the prepared second composition can effectively supplement and maintain high plasma concentrations of xanthine derivatives at a later stage of ingestion.

Referring to Preparation Example B4 (Ex4), it also has an acceptable controlled release effect. As shown in FIG. 5 and Table 5, the in vitro dissolution of the composition of Preparation Example B4 releases less than 35% by weight of the xanthine derivative (caffeine) within 90 minutes, and releases less than 40% by weight of the xanthine derivative (caffeine) within 2 hours. Therefore, the second composition of Preparation Example B4 can also be controlled to delay release at a later stage of ingestion (after 2 hours), which can facilitate the supplement and maintenance of high plasma concentrations of xanthine derivatives. As shown in Table 2 above, a weight proportion of the zein enteric coating in Preparation Examples B1, B2 and B4 are controlled in the range of 5.5% by weight to 10% by weight of the second composition. Therefore, the controlled-release effect of the present invention can be achieved.

In comparison, referring to Preparation Example B3 (Ex3), when a weight proportion of the zein enteric coating as the enteric excipient is 3% by weight of the second composition (as shown in Table 2), it cannot effectively achieve the controlled-release effect. As illustrated in FIG. 5, the release of xanthine derivative (caffeine) in the in vitro dissolution of the composition of Preparation Example B3 has exceeded 40% by weight within 90 minutes, which is similar to the dissolution profile of conventional sustained release powder formulations. Therefore, the second composition of Preparation Example B3 cannot effectively achieve the controlled-release effect at specific time periods.

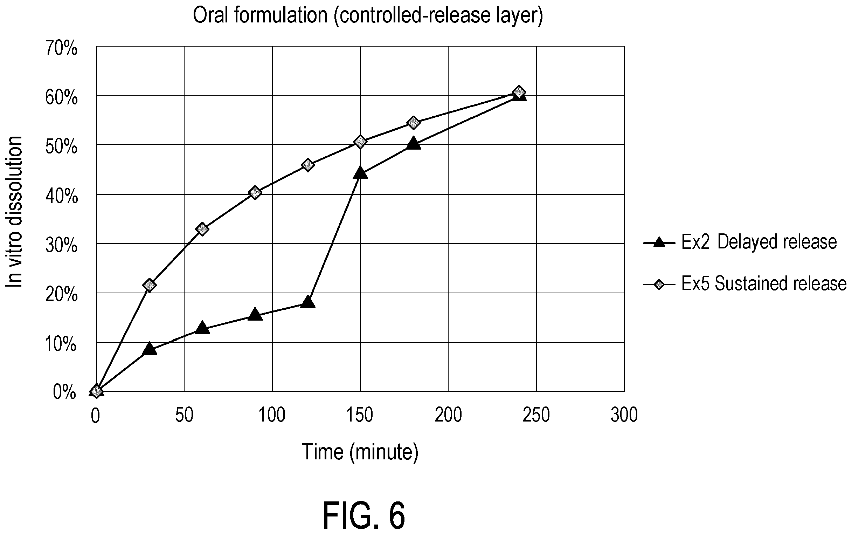

To further confirm the difference between the enteric granulation of the present invention and the conventional matrix type granulation, the test results of Preparation Example B2 (Ex2) and Preparation Example C5 (Ex5) are individually compared. FIG. 6 is an in vitro dissolution profile comparison diagram of an enteric granulation of the present invention (Ex2) and a conventional matrix type granulation (Ex5). Preparation Example B2 (Ex2) and Preparation Example C5 (Ex5) have the same proportion of zein enteric coating and green tea extracts (both 10% by weight and 71.43% by weight). However, Preparation Example B2 was prepared by enteric granulation (steps in FIG. 3), while Preparation Example C5 was prepared by matrix mixing (steps in FIG. 4), and these two preparation methods are substantially different.

Referring to FIG. 6, it is noted that even if the same weight proportion of zein is used for granulation, if the powder was not prepared by the enteric granulation steps of the present invention, then the controlled-release effect cannot be effectively achieved. As shown in FIG. 6, the release of xanthine derivative (caffeine) in the in vitro dissolution of the Preparation Example C5 has reached 40% by weight within 90 minutes, and thus it is a sustained release dosage form. In comparison, the release of xanthine derivative (caffeine) in the in vitro dissolution of the Preparation Example B2 of the present invention is less than 20% by weight within 2 hours, and the release of xanthine derivative (caffeine) reached 46% by weight within 150 minutes. Thus, an obvious effect of delayed release under control can be achieved.

### In vitro dissolution test II

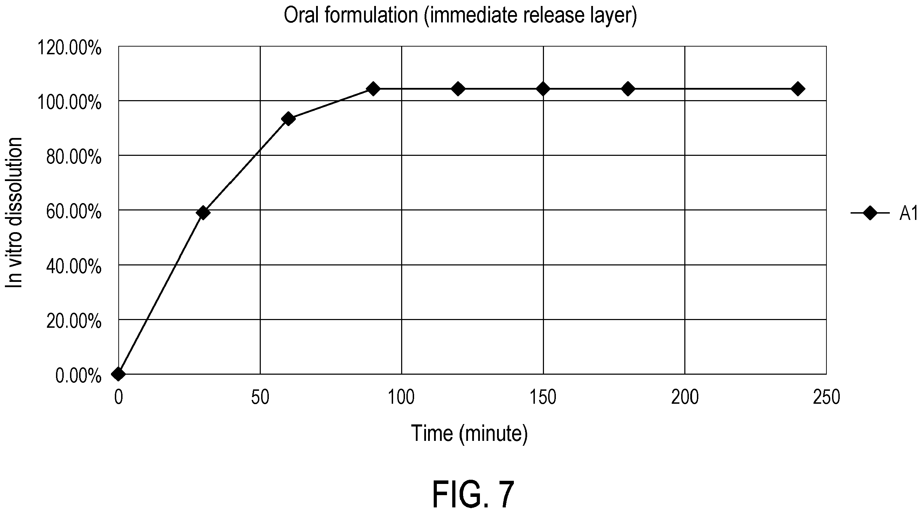

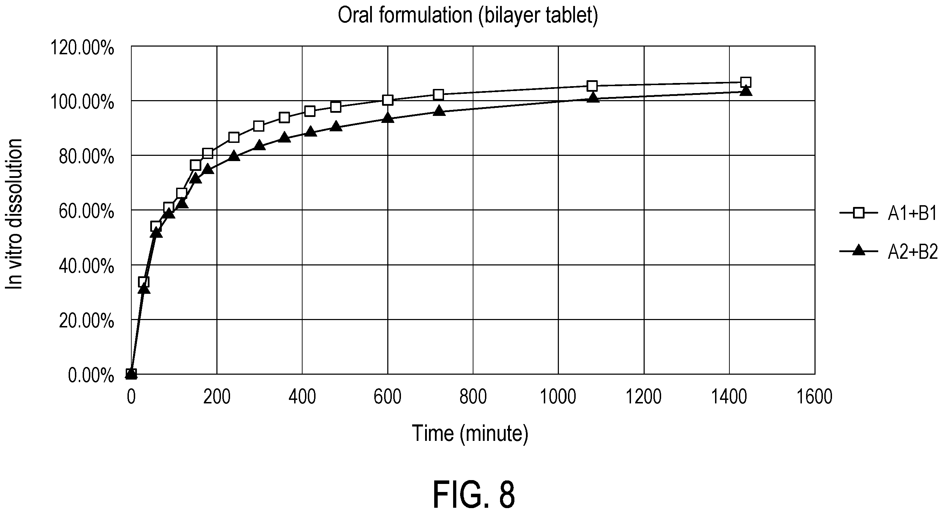

To confirm that the first composition of the present invention has an immediate release effect, the first composition of Preparation Example A1 was used to conduct an in vitro dissolution test. In addition, to confirm the dissolution performance of the oral formulation 100 of the present invention, oral formulations of bilayer tablet was prepared by combining the first composition of Preparation Example A1 with the second composition of Preparation Example B1 (A1+B1), and the first composition of Preparation Example A2 with the second composition of Preparation Example B2 (A2+B2), with which the in vitro dissolution tests are performed. The steps of the in vitro dissolution test are similar to those mentioned in "In vitro dissolution test I", and thus will not be repeated herein. The difference being that in the dissolution test of the oral formulation (bilayer tablet), the collection of the solution is continued until 1440 minutes (24 hours). The test results are shown in FIG. 7 and FIG. 8.

FIG. 7 is an in vitro dissolution profile diagram of a first composition (immediate release layer) according to an embodiment of the present invention. Referring to FIG. 7, it can be seen that the first composition prepared by Preparation Example A1 of the present invention has an obvious immediate release effect. As shown in the test results in FIG. 7, the in vitro dissolution release of xanthine derivative (caffeine) has reached more than 80% within 60 minutes, and the in vitro dissolution release of xanthine derivative (caffeine) has reached 100% within 90 minutes. Accordingly, the first composition of the present invention can effectively release the xanthine derivative rapidly at an early stage (within 2 hours) after ingestion, maintain a high plasma concentration, and provide an ergogenic effect.

FIG. 8 is an in vitro dissolution profile diagram of an oral formulation according to an embodiment of the present invention. Referring to FIG. 8, in both of the oral bilayer tablet formed by the combination of Preparation Example A1 and Preparation Example B1 and the oral l bilayer tablet formed by the combination of Preparation Example A2 and Preparation Example B2, the in vitro dissolution release of the xanthine derivative (caffeine) was shown to be 50% or more within 90 minutes. In other words, the first composition of the oral bilayer tablet has been fully released, while the second composition is controlled to be slowly released. In addition, within 90 minutes to 720 minutes (12 hours) of in vitro dissolution, the release of the xanthine derivative (caffeine) will increase to 90% or more, and the release of the xanthine derivative (caffeine) will reach 100% by weight within 12 to 18 hours of the in vitro dissolution. Accordingly, through the immediate release of the first composition and the controlled release of the second composition, the bilayer controlled-release oral formulation of the present invention can effectively control the release of the xanthine derivative (such as caffeine) to maintain high plasma concentrations, thereby satisfying long term ergogenic/stimulation demands.

In summary, the embodiments of the present invention utilize an oral formulation having a first composition and a second composition, and the second composition has an enteric excipient prepared by enteric granulation. Therefore, the oral formulation of the present invention can immediately release the first composition at an early stage after ingestion, and provide an early caffeine supplementation, and controllably release the second composition of caffeine at a later stage. Since the second composition includes an edible enteric coating material, it can gradually dissolve in an alkaline intestinal environment, which results in a second stage of release. Accordingly, the bilayer controlled-release oral formulation of the present invention can effectively control the release of the xanthine derivatives (such as caffeine) to maintain high plasma concentrations, thereby meeting long-term ergogenic/stimulation needs. For example, the bilayer controlled-release oral formulation of the present invention can resolve the necessities of repeated supplementations/stimulations during prolonged activities, such as during endurance sports like marathons where runners need to take additional caffeine products for performance enhancement, or situations like long distance driving where inconvenience and distraction can be caused by repeated supplementation. Furthermore, through the controlled-release of the second composition, the high content/plasma concentration of caffeine can be maintained throughout the entire exercising period, and it can also continue to provide the ergogenic/stimulation effect after exercise.

## Claims

1. An oral formulation, comprising:
a first composition, comprising a xanthine derivative; and
a second composition, comprising a xanthine derivative and an enteric excipient, wherein an in vitro dissolution of the second composition releases less than 35% by weight of the xanthine derivative in the second composition within 90 minutes, and the in vitro dissolution of the second composition releases 50% by weight or more of the xanthine derivative in the second composition within 3 hours.

2. The oral formulation according to claim 1, wherein the in vitro dissolution of the second composition releases 50% by weight or more to 70% by weight or less of the xanthine derivative in the second composition within 3 hours.

3. The oral formulation according to claim 1, wherein in vitro dissolution of the first composition releases 80% by weight or more of the xanthine derivative in the first composition within 1 hour.

4. The oral formulation according to claim 1, wherein the in vitro dissolution of the first composition releases up to 100 % by weight of the xanthine derivative in the first composition within 90 minutes, the in vitro dissolution of the second composition releases 90% or more by weight of the xanthine derivative in the second composition within 8 hours to 12 hours, and the in vitro dissolution of the second composition releases up to 100 % by weight of the xanthine derivative of the second composition within 12 hours to 18 hours.

5. The oral formulation according to claim 1, wherein the in vitro dissolution of the second composition releases less than 40% by weight of the xanthine derivative of the second composition within 2 hours.

6. The oral formulation according to claim 1, wherein the oral formulation is an oral bilayer tablet, which comprises a first layer composed of the first composition, and a second layer composed of the second composition.

7. The oral formulation according to claim 1, wherein a weight ratio of the first composition to the second composition is 1:1 to 1:1.2.

8. The oral formulation according to claim 1, wherein the enteric excipient is a zein enteric coating.

9. The oral formulation according to claim 8, wherein when a weight proportion of the second composition is 100%, a weight proportion of the zein enteric coating is 5.5% to 10% of the second composition.

10. The oral formulation according to claim 1, wherein a total weight of the xanthine derivative in the oral formulation is 50mg to 400mg.

11. The oral formulation according to claim 10, wherein a total weight of the xanthine derivative in the first composition is 25mg to 200mg.

12. The oral formulation according to claim 10, wherein a total weight of the xanthine derivative in the second composition is 25mg to 200mg.

13. The oral formulation according to claim 1, wherein the first composition further comprises green tea extract, polyvinylpyrrolidone, microcrystalline cellulose, crospovidone, magnesium stearate and silicon dioxide, wherein the green tea extract includes the xanthine derivative.

14. The oral formulation according to claim 1, wherein the second composition further comprises green tea extract, polyvinylpyrrolidone, microcrystalline cellulose, medium chain triglyceride, magnesium stearate, silicon dioxide and coating 145K290001, wherein the green tea extract includes the xanthine derivative.

15. The oral formulation according to claim 1, wherein the oral formulation reaches the highest plasma concentration of the xanthine derivative within 90 minutes of ingestion.

16. A preparation method of the oral formulation of claim 1, wherein the method comprises:
forming a bilayer tablet with a powder of the first composition and a powder of the second composition.

17. The preparation method according to claim 16, wherein a preparation of the powder of the second composition comprises:
providing a coating suspension including the enteric excipient;
providing a green tea extract, wherein the green tea extract includes the xanthine derivative; and
spray granulating the green tea extract with the coating suspension so as to gain weight in a range of 5% to 15%.

18. The preparation method according to claim 17, wherein the method further comprises:
after the spray granulating, performing drying and sieving to form the powder of the second composition.

19. The preparation method according to claim 16, wherein a preparation of the powder of the first composition comprises:
providing a green tea extract, wherein the green tea extract includes the xanthine derivative; and
adding an alcohol aqueous solution of polyvinylpyrrolidone to a powder including the green tea extract, and after granulating, performing drying and sieving to form the powder of the first composition.

20. A method of using the oral formulation according to claim 1, wherein the method comprises:
ingesting the oral formulation 30 minutes to 60 minutes before performing endurance exercise, and a dosage of the oral formulation is 3mg/kg to 6mg/kg.
